# EUROPEAN PATENT SPECIFICATION

(11) **EP 3 017 815 B1**
(45) Date of publication and mention of the grant of the patent: **09.12.2020**
(21) Application number: 14818368.4
(22) Date of filing: 24.01.2014
(51) Int. Cl.: A61K 31/352, A23L 33/00, A61P 35/00, A61P 9/00, A61P 29/00, A23L 33/105

(54) **USE OF NARINGENIN IN PREPARING DRUGS FOR PREVENTING AND/OR TREATING ABDOMINAL AORTIC ANEURYSM**
VERWENDUNG VON NARINGENIN BEI DER HERSTELLUNG VON ARZNEIMITTELN ZUR PRÄVENTION UND/ODER BEHANDLUNG VON BAUCHAORTENANEURYSMEN
UTILISATION DE NARINGÉNINE POUR PRÉPARER DES MÉDICAMENTS POUR PRÉVENIR ET/OU TRAITER UN ANÉVRISME AORTIQUE ABDOMINAL

(30) Priority: 27.06.2013 CN 201310264289
(43) Date of publication of application: 11.05.2016
(73) Proprietor: Peking University, Beijing 100191 (CN)
(72) Inventor: KONG, Wei, Beijing 100191 (CN); CUI, Qinghua, Beijing 100191 (CN); LIU, Ziyi, Beijing 100191 (CN); YU, Fang, Beijing 100191 (CN); ZHENG, Jingang, Beijing 100029 (CN)
(74) Representative: Graf von Stosch, Andreas
(86) International application number: PCT/CN2014/000093
(87) International publication number: WO 2014/206056

(56) References cited:
- CN-A- 102 302 483
- CN-A- 103 340 849
- US-A1- 2010 278 914
- M.J. DAVIES: "Aortic Aneurysm Formation : Lessons From Human Studies and Experimental Models", CIRCULATION, vol. 98, no. 3, 21 July 1998 (1998-07-21), pages 193-195, XP055330645, US ISSN: 0009-7322, DOI: 10.1161/01.CIR.98.3.193
- R. LIM ET AL: "Dietary phytophenols curcumin, naringenin and apigenin reduce infection-induced inflammatory and contractile pathways in human placenta, foetal membranes and myometrium", MOLECULAR HUMAN REPRODUCTION., vol. 19, no. 7, 26 March 2013 (2013-03-26) , pages 451-462, XP055330684, GB - BE ISSN: 1360-9947, DOI: 10.1093/molehr/gat015
- DASTMALCHI ET AL: "Chemical composition and in vitro antioxidative activity of a lemon balm (Melissa officinalis L.) extract", LWT- FOOD SCIENCE AND TECHNOLOGY, ACADEMIC PRESS, UNITED KINGDOM, vol. 41, no. 3, 27 November 2007 (2007-11-27), pages 391-400, XP022365063, ISSN: 0023-6438, DOI: 10.1016/J.LWT.2007.03.007
- SIYU CHEN ET AL.: 'Naringenin inhibits TNF- a induced VSMC proliferation and migration via induction of HO-1.' FOOD AND CHEMICAL TOXICOLOGY. vol. 50, 2012, pages 3025 - 3031, XP055306776
- T MIYAKE ET AL.: 'Prevention of abdominal aortic aneurysms by simultaneous inhibition of NFkB and ets using chimeric decoy oligonucleotides in a rabbit model.' GENE THERAPY. vol. 13, 2006, pages 695 - 704, XP055306777

## Description

### Technical Field

The present application relates to use of naringenin in the pharmaceutical field.

### Background

An abdominal aortic aneurysm (AAA) is a cardiovascular disease with high lethality. It refers to a permanent expansion of an abdominal aortic wall, and if the expansion of the wall is greater than 50% of diameter of the adjacent site, or the diameter thereof exceeds 3 cm, it can be considered to suffer from an abdominal aortic aneurysm (Johnston KW, Rutherford RB, Tilson MD, Shah DM, Hollier L, Stanley JC. Suggested standards for reporting on Subcommittee on Reporting Standards for Arterial Aneurysms, Ad Hoc Committee on Reporting Standards, Society for Vascular Surgery and North American Chapter, International Society for Cardiovascular Surgery. J Vasc Surg. 1991;13(3):452-458). Once the aneurysmal aorta ruptures, the patient will die from massive blood loss, therefore, an abdominal aortic aneurysm is also called as "the time bomb in the human body". This disease usually happens in older people, especially in male. The incidence gradually rises with age, and can be up to 8-10%, and the fatality rate reaches to 50% -80%. In USA, the death caused by rapture of an abdominal aortic aneurysm ranks fifteenth in all causes of death and tenth in causes of death of adult male diseases. While in PRC, in the recent 30 years, the incidence thereof rapidly rises by 3 times. However, the pathogenesis and risk factors about this disease are not very clear up to now, therefore, there is no effective method for early prevention, early warning, and drug prevention and treatment. Since DuBost firstly successfully implemented abdominal aortic aneurysm resection and artificial vascular graft at 1951 (DuBost C, Allary M, Oeconomos N. Resection of an aneurysm of the abdominal aorta: reestablishment of the continuity by a preserved human arterial graft, with result after five months. AMA Arch Surg. 1952 Mar;64(3):405-8), with the continuous development and improvement of technologies, an endovascular repair has been widely accepted and used globally, and becomes a most commonly used method for treating an abdominal aortic aneurysm at present. However, there are many disadvantages in the surgery, such as high-risk, expensive, not a permanent cure, and difficult to popularize. And as for the patient having a small abdominal aortic aneurysm, that is, when the aneurysmal aorta diameter is less than 5.5 cm, the risk of rupture may be less than the risk of surgery. However, there is no effective drug which can prevent and treat an abdominal aortic aneurysm at present, therefore, exploring a prevention and treatment method for the treatment of abdominal aortic aneurysms now has become a focus and hotspot for cardiovascular research.

For example, Davies reported that aortic aneurysms are primarily caused by an inflammatory response within the blood vessel walls, which leads to destruction of the connective tissue matrix caused by proteolytic digestion, involving, for example, matrix metalloproteinases (Davies MJ. Aortic aneurysm formation: lessons from human studies and experimental models. Circulation. 1998 Jul 21;98(3):193-5).

Research shows that the pathogenesis of an abdominal aortic aneurysm is mainly due to rise in reactive oxygen species (ROS) of vascular walls, increase in infiltration of inflammatory cells and release of inflammatory cytokines, rise in activities of matrix metalloproteinases (MMP), causing inflammatory response of vascular walls and significant increase of matrix degradation, thereby to result in the onset of abdominal aortic aneurysms (Daugherty A, Cassis LA. Mouse models of abdominal aortic aneurysms. Arterioscler Thromb Vasc Biol. 2004 Mar; 24(3):429-34). This suggests if drugs for inhibiting vascular inflammatory response or reducing matrix degradation are used, onset and development of abdominal aortic aneurysms may be alleviated or treated. At present, no specific drug treatment schemes supported by large-scale clinical randomized control studies are available. However, there are small-scale clinical trials for some drugs for treating abdominal aortic aneurysms, such as statins (Schouten D et al. Statins are Associated with a Reduced Infrarenal Abdominal Aortic Aneurysm Growth. Eur J Vasc Endovasc Surg.2006 32, 21-26.), macrolide antibiotics (Vammen S et al. Randomized double-blind controlled trial of roxithromycin for prevention of abdominal aortic aneurysm expansion. Br J Surg 2001 88(8):1066-1072), adrenergic receptor blockers (Gregory R et al. Abdominal aortic aneurysm expansion rate: Effect of size and beta-adrenergic blockade. J Vasc Surg.1994 Apr;19(4):727-31), and doxycycline (Mosorin M et al. Use of doxycycline to decrease the growth rate of abdominal aortic aneurysms: a randomized, double-blind, placebo-controlled pilot study. J Vasc Surg 2001 34(4):606-610), and they all have a certain therapeutic effect on patients having abdominal aortic aneurysms. In addition, an inhibitor of prostaglandin synthase (Walton LJ et al. Inhibition of prostaglandin E2 synthesis in abdominal aortic aneurysms: implications for smooth muscle cell viability, inflammatory processes, and the expansion of abdominal aortic aneurysms. Circulation.1999 100(1):48-54) and an anti-inflammatory drug, indomethacin, (Holmes DR et al. Indomethacin prevents elastase-induced abdominal aortic aneurysms in the rat. J Surg Res. 1996 Jun;63(1):305-9.) also have a certain effect in animal models in which abdominal aortic aneurysms are induced. Most of these drugs are indicated to reduce the inflammatory response of blood vessels, or inhibit the activities of metalloproteinases, thereby to function. However, the above drugs all lack evidence of large-scale randomized controlled clinical studies supporting effectiveness thereof. When applied to clinical trials, some drugs, such as doxycycline, also produce frequent side effects (Baxter BT et al. Prolonged administration of doxycycline in patients with small asymptomatic abdominal aortic aneurysms: report of a prospective (Phase II) multicenter study. J Vasc Surg. 2002 Jul; 36(1):1-12). Therefore, it will have enormous clinical application value to develop a new drug possessing good prevention and treatment effects and few side effects for treating abdominal aortic aneurysms.

US 2012/278914 A1 discloses the use of a Melissa ethyl acetate fraction as an angiogenesis and MMP inhibitor for various diseases including abdominal aortic aneurysms. Dastmalchi et al. showed that Melissa leaf extracts obtained by a distinct method, namely, aqueous ethanol extraction, contain low amounts of naringenin among various other compounds extracted in much larger quantities (Keyvan Dastmalchi, H.J. Damien Dorman, Päivi P. Oinonen, Yusrida Darwis, Into Laakso, Raimo Hiltunen, Chemical composition and in vitro antioxidative activity of a lemon balm (Melissa officinalis L.) extract, LWT - Food Science and Technology, Volume 41, Issue 3, 2008, Pages 391-400). Lim et al. relates to the role of dietary naringenin on infection-induced premature birth (Ratana Lim, Gillian Barker, Courtney A. Wall, Martha Lappas, Dietary phytophenols curcumin, naringenin and apigenin reduce infection-induced inflammatory and contractile pathways in human placenta, foetal membranes and myometrium, Molecular Human Reproduction, Volume 19, Issue 7, July 2013, Pages 451-462).

Naringenin is a natural flavonoid compound, which is a highly expressed flavonoid in citrus plants such as grapefruit, shaddock peel, orange peel, and potato peel, and in traditional Chinese medicine, such as Huyou peel, phellinus igniarius, and Ilex centrochinensis. In animal experiments, it has been reported that naringenin can reduce blood fat, and reduce the levels of cholesterol, triglycerides, and low density lipoprotein (LDL-C) in serum, thereby reducing onset and development of atherosclerosis (Mulvihill E, Assini J, Sutherland B, DiMattia A, Khami M, Koppes J, Sawyez C, Whitman S, Huff M. in High-Fat -Fed Low-Density Lipoprotein Receptor-Null Mice Naringenin Decreases Progression of Atherosclerosis by Improving Dyslipidemia. Arterioscler Thromb Vasc Biol 2010, 30:742-748). It also can regulate immune function and inhibit tumor growth (Lou C, Zhang F, Yang M, Zhao J, Zeng W, Fang X, Zhang Y, Zhang C, Liang W. Naringenin Decreases Invasiveness and Metastasis by Inhibiting TGF-β-Induced Epithelial to Mesenchymal Transition in Pancreatic Cancer Cells. PLoS ONE 2012;7(12).). In addition, naringenin also can treat insulin resistance and Type II diabetes (Mulvihill EE, Allister EM, Sutherland BG, Telford DE, Sawyez CG, Edwards JY, Markle JM, Hegele RA, Huff MW. Naringenin prevents dyslipidemia, apolipoprotein B overproduction, and hyperinsulinemia in LDL receptor-null mice with diet-induced insulin resistance. Diabetes.2009;58(10):2198-210), as well as hepatopathy (Morcos PN, Brennan B, Smith PF. A grapefruit a day for patients infected with hepatitis C Hepatology. 2008 Jun;47(6):2141-2) in animal models. However, there are no relevant reports about use of naringenin for prevention and treatment of the onset of abdominal aortic aneurysms.

### Summary

The purpose of the present invention is to provide a new use of naringenin as a drug.

The present invention provides naringenin for use in a method of preventing and/or treating abdominal aortic aneurysms.

In particular, said use of naringenin also can comprise at least one of the following:
1) inhibiting the fracture of abdominal aorta elastic lamina;
2) inhibiting the infiltration of inflammatory cells of abdominal aorta vascular walls; the inflammatory cells can be macrophages and/or other leukocytes;
3) inhibiting the levels of IL-6 and MCP-1 in plasma;
4) inhibiting the expression of inflammatory factors in abdominal aorta vascular walls; the inflammatory factors can be interleukin IL-6 and/or monocyte chemotactic factor MCP-1;
5) inhibiting the activity and expression of matrix metalloproteinase of abdominal aorta vascular walls; the matrix metalloproteinase specifically can be MMP-9.

The drugs prepared by employing naringenin as an active ingredient for preventing and/or treating abdominal aortic aneurysms also fall into the protection scope of the present invention.

The drugs can be introduced into the body such as muscle, intracutaneous, subcutaneous, vein, and mucosal tissues via the methods of injection, spraying, nasal drip, eye drip, permeation, absorption, or physical or chemical intervention; or they can be introduced into the body after they are mixed with other substances or encapsulated by other substances.

When needed, one or more pharmaceutically acceptable carriers can be added to the above drugs. The carriers comprise conventional diluents, excipients, fillers, binders, humectants, disintegrating agents, absorption enhancers, surfactants, adsorption carriers, lubricants.

The drugs can be prepared into various forms such as injections, suspending agents, powders, tablets and granules. The above various dosage forms of drugs can be prepared according to conventional methods of the pharmaceutical field.

The present invention uses two clinically associated abdominal aortic aneurysm disease models: using angiotensin II (1000 ng/kg/min) through subcutaneous implanted pump to induce ApoE knockout mice; and using calcium phosphate (0.5 M) to daub the abdominal aortas of C57 mice locally induced with abdominal aortic aneurysms. Experiments demonstrate that naringenin via oral gavage can significantly inhibit the onset and development of an abdominal aortic aneurysm in mice daubed with CaPO₄ and implanted with Angll pumps, and thereby naringenin can be used in the treatment of patients with abdominal aortic aneurysms.

The drugs for preventing and/or treating abdominal aortic aneurysms provided by the present invention are safe and low toxic, and the pharmacologic effects thereof are strong; sources of the raw materials thereof are rich, wide, inexpensive, and it can be obtained from crude products or from monomers of naringin by a hydrolysis method, or it can be obtained by extracting it from various crude medicines containing naringenin, or it can be obtained by using other chemical synthesis methods; the costs are low, the process is simple, and the yield is high; the curative effect is definite, and the effective dose is low. A new drug source for preventing, diagnosing, detecting, protecting, treating, and studying abdominal aortic aneurysm diseases is described herein, which is easy to popularize and apply, and which can produce huge social and economic benefits within a short period of time.

### Brief Description

Figure 1 is a series of vascular photos showing the reduction of the onset of abdominal aortic aneurysms by naringenin (NGN) gavage in C57BL/6J mice with CaPO₄ daubed infrarenal aortas, wherein doxycycline (Dox) is used as a positive control of animal experiments.
Figure 2 shows the reduction of the severity of abdominal aortic aneurysms by naringenin (NGN) gavage in C57BL/6J mice with CaPO₄ daubed infrarenal arteries, wherein doxycycline is used as a positive control of animal experiments.
   A shows the diameters of infrarenal arteries, identified by histological staining, of C57BL/6J mice with NaCl daubing and feeding water, CaPO₄ daubing and feeding water, naringenin or doxycycline and the statistical situation thereof; and
   B shows the fracture degree of infrarenal arterial elastic lamina, identified by histological staining, of C57BL/6J mice with NaCl daubing and feeding water, CaPO₄ daubing and feeding water, naringenin or doxycycline and the statistical situation thereof.

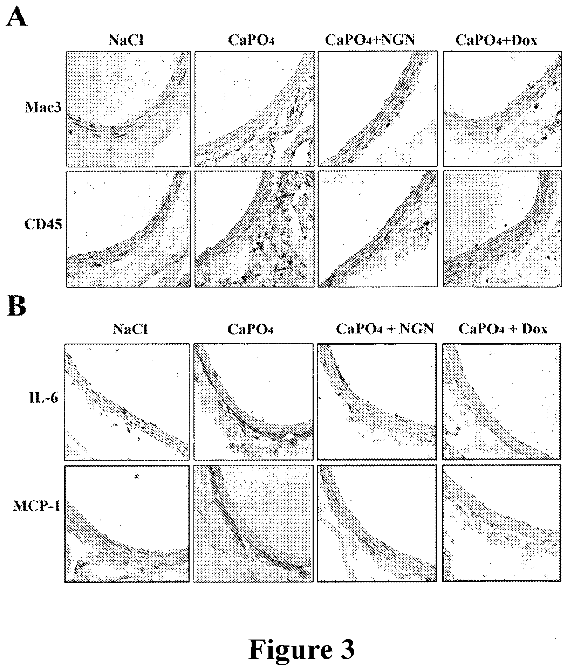

Figure 3 shows the reduction of the inflammatory response of infrarenal arterial vessel in CaPO₄ daubed C57BL/6J mice by naringenin or doxycycline;
   A shows the reduction of the inflammatory cell (macrophage and leukocyte) infiltration in vascular walls of infrarenal arteries, identified by immunohistochemistry, of C57BL/6J mice receiving a naringenin or doxycycline gavage; and
   B shows the reduction of the inflammatory factor (interleukin IL-6/monocyte chemotatic factor MCP-1) expression in vascular walls of infrarenal arteries, identified by immunohistochemistry, of C57BL/6J mice receiving a naringenin or doxycycline gavage.

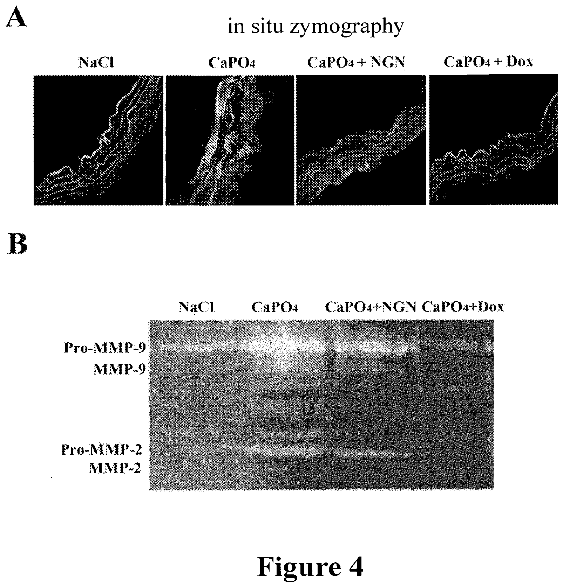

Figure 4 shows the inhibition of the activities and expression of matrix metalloproteinases by naringenin or doxycycline in the vascular walls of infrarenal arteries of CaPO₄ daubed C57BL/6J mice;
   A shows the inhibition of the activity of CaPO₄ induced matrix metalloproteinase MMP with naringenin or doxycycline validated by *in situ* zymography; and
   B shows the inhibition of the activities of CaPO₄ induced MMP-2 and MMP-9 with naringenin or doxycycline validated by gelatin zymography.

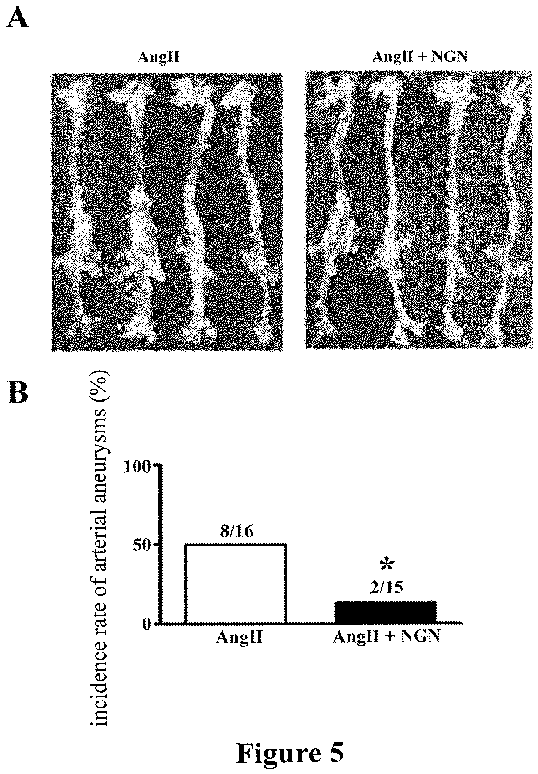

Figure 5 shows the reduction of the onset of abdominal aortic aneurysms by a naringenin gavage in angiotensin II induced ApoE^{-/-} mice;
   A is a series of vascular photos showing the onset situation of abdominal aortic aneurysms in ApoE^{-/-} mice which are gavaged with naringenin; and
   B is a statistical graph showing the morbidity of abdominal aortic aneurysms in ApoE^{-/-} mice which are gavaged with naringenin.

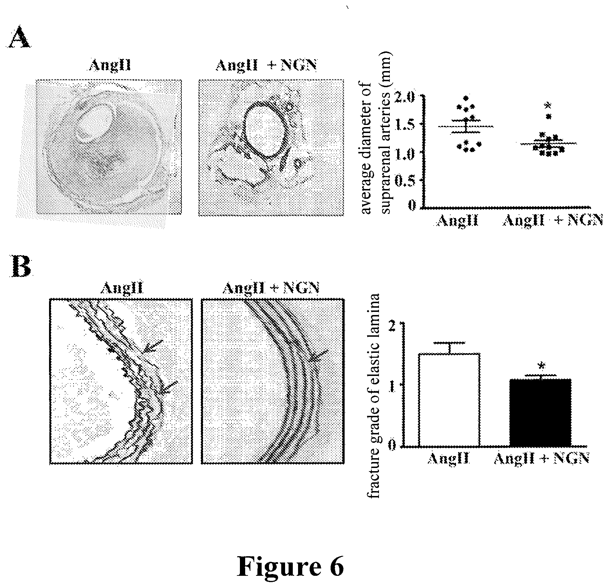

Figure 6 shows the reduction of the severity of abdominal aortic aneurysms in angiotensin II induced ApoE^{-/-} mice.
   A shows the maximum diameter of suprarenal aortas, identified by histological staining, of mice which are gavaged with naringenin or water and the statistical situation thereof; and
   B shows the fracture degree of elastic lamina of suprarenal aortas, identified by histological staining, of mice which are feed on naringenin or water and the statistical situation thereof.

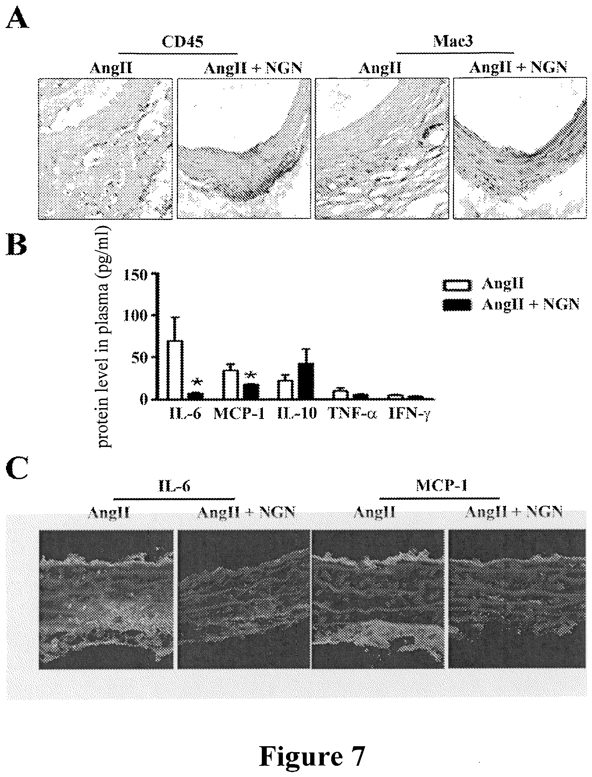

Figure 7 shows the reduction of the inflammation degree of vascular walls of suprarenal aortas by a naringenin gavage in angiotensin II induced ApoE^{-/-} mice;
   A shows the reduction of inflammatory cell infiltration in vascular walls, identified by immunohistochemistry, of angiotensin II induced ApoE^{-/-} mice with a naringenin gavage;
   B shows the reduction of the expressions of inflammatory factors in the plasma of angiotensin II induced ApoE^{-/-} mice by a naringenin gavage; and
   C shows the reduction of the release of inflammatory factors in vascular walls of mice by a naringenin gavage.

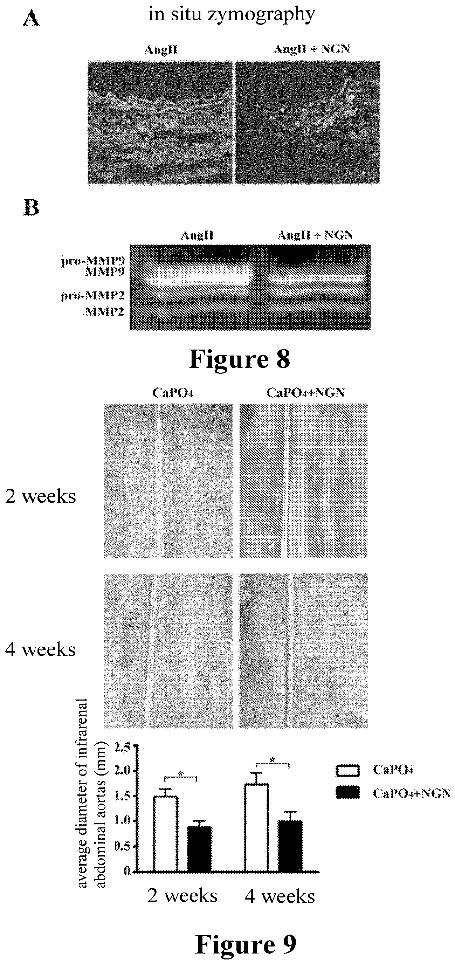

Figure 8 shows the reduction of the activities of matrix metalloproteinases in vascular walls of suprarenal arteries by a naringenin gavage in angiotensin II induced ApoE^{-/-} mice;
   A shows the inhibition of the activities of angiotensin II induced matrix metalloproteinases with naringenin validated by *in situ* zymography;
   B shows the inhibition of the activity of angiotensin II induced MMP-2 and MMP-9 with naringenin validated by gelatin zymography.
Figure 9 shows a series of figures of abdominal aortas and a statistical graph of diameters thereof measured ex vivo in 12-week old male C57 mice in Example 5, wherein the mice were daubed with CaPO₄ for 7 days, and then were gavaged with naringenin (50 mg/kg/day) and water for 2 weeks and 4 weeks, respectively.

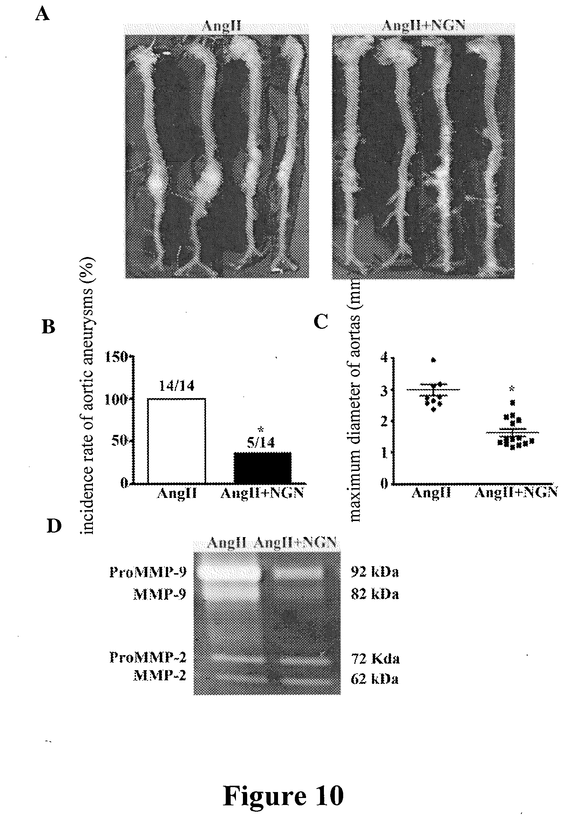

Figure 10 is a series of figures of 20-weeks old male ApoE knockout mice in Example 5, wherein the mice received angiotensin II implanted pump treatment for 28 days and an ultrasonic testing was performed, and the mice in which aneurysms had formed were equally divided into two groups to feed on naringenin or water, and received another angiotensin II implanted pump treatment for 28 days;
   A is a series of vascular photos showing the onset of aneurysms in mice after the second round of angiotensin II treatment;
   B shows incidence rate % of ApoE knockout mice in which aneurysms previously have formed after the second round of angiotensin II implanted pump treatment, relative to the number of mice in which aneurysm previously had formed;
   C is a quantification figure of the maximum diameter of abdominal aortas of mice via ultrasonic testing; and
   D shows the effect of naringenin on the activities of metalloproteinases MMP-9 and MMP-2 of suprarenal abdominal aortas of ApoE knockout mice, detected by gelatin zymography; each group of 3 mice.

### Detailed description

Unless expressly indicated otherwise, the experimental methods described in the following Examples are all conventional methods; and unless expressly indicated otherwise, all the reagents and biological materials can be commercially available.

Example 1: the reduction of the onset of abdominal aortic aneurysms by a naringenin gavage in C57BL/6J mice with CaPO₄ daubed infrarenal arteries

1. Models of abdominal aortic aneurysms induced by CaPO₄ daubed infrarenal aortas of mice

12-week old of male C57BL/6 mice were anesthetized via intraperitoneal injection of 0.1% pentobarbital sodium (injection according to body weight, 6-7µl/g). Infrarenal abdominal aortas were separated, and the separated infrarenal abdominal aortas were packaged by gauze (1 cm in width x 3 cm in length) soaked in 0.5 M CaCl₂ solution. After 10 minutes, the gauze was removed, gauze was soaked in PBS instead and used to package the blood vessel for 5 min. CaCl₂+and PO₄⁻³ in PBS can form CaPO₄ crystals, that is, corresponding to daubing CaPO₄ to the blood vessel. After packaging the blood vessel with gauze soaked in NaCl for 10 minutes, gauze soaked in PBS is used instead for 5 min, serving as NaCl daubed control group.

### 2. Models of naringenin (NGN) or doxycycline (Dox) gavage Doxycycline was used as a positive control drug.

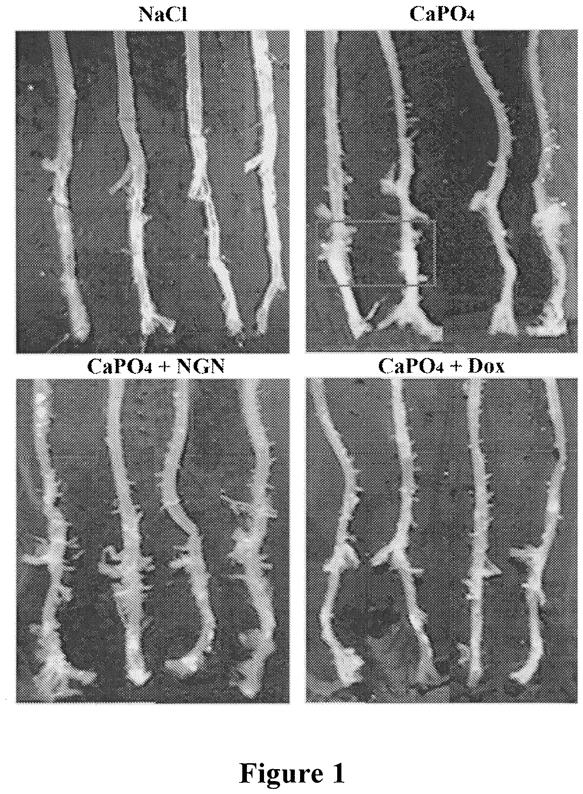

Gavage was started at the first day after surgery, and water, naringenin (50 mg/kg) (Sigma-Aldrich, St. Louis, MO) or doxycycline (100 mg/kg) (Sigma-Aldrich, St. Louis, MO) gavage were given to mice daily. Materials were removed from the mice 7 days after gavage. Mice were weighed, and blood was drawn from inner canthus by capillary, and after centrifuging for 10 minutes at 6000 rotations, the supernatant was removed and blood fat was measured (kit purchased from BIOSINO company). Then perfusion and washing were performed on mice using PBS. Blood vessels were separated, and four blood vessels were randomly chosen for each group for photographing. The results were shown in Figure 1, wherein NaCl was the group of daubing NaCl (n=11), CaPO₄ was the group of daubing CaPO₄ plus water feed (n=12), CaPO₄+NGN was the group of daubing CaPO₄ plus naringenin gavage (n=11), and CaPO₄+Dox was the group of daubing CaPO₄ plus doxycycline gavage (n=11). The results demonstrate that, compared with mice in the group of daubing NaCl, the blood vessels at infrarenal site in CaPO₄ daubed mice exhibit significant bulge, while a naringenin or doxycycline gavage can reduce the incidence rate and degree of bulge.

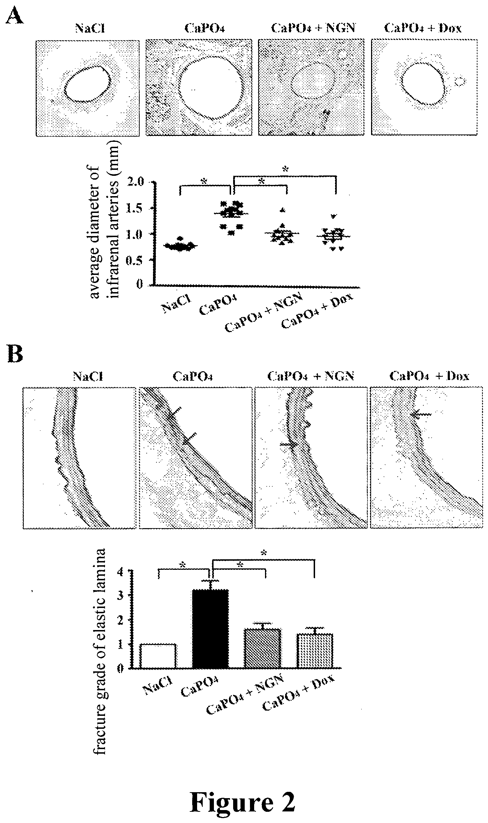

The blood vessels were sliced, and the change of the diameters in infrarenal abdominal aortas of mice were identified by H&E staining. The results in Figure 2A show that CaPO₄ daubing can significantly increase the diameters of infrarenal blood vessels of mice, compared with the group of daubing NaCl (NaCl *vs.* CaPO₄, 0.77±0.06 *vs.* 1.40±0.19 mm, *P*<0.05). However, a naringenin or doxycycline gavage daily can significantly reduce the hemangiectasis caused by CaPO₄ daubing, and the effects of naringenin and doxycycline are similar (CaPO₄ + NGN *vs.* CaPO₄ + Dox, 1.03±0.18 *vs.* 0.99±0.18 mm). The data in Figure 2A were an average value ± standard deviation.

The fracture degree of the elastic lamina is also an index showing the severity of abdominal aortic aneurysms. The degradation of the elastic lamina is divided into four grades. The first grade means that the degradation of the elastic lamina is less than 25%, with the second grade relating to 25%-50%, the third grade relating to 50%-75%, and the fourth grade meaning that the fracture is more than 75%. We used Gömöri staining to identify the fracture situation of the elastic lamina of infrarenal abdominal aortas of mice. The results in Figure 2B show that, compared with NaCl daubing, CaPO₄ daubing significantly increases the fracture situation of the elastic lamina of infrarenal abdominal aortas of mice, while a naringenin or doxycycline gavage can reverse the effect of CaPO₄. The data in Figure 2B were an average value ± standard deviation.

In addition, the naringenin gavage does not affect the body weights and blood fat levels of C57BL/6J mice, see Table 1.

**Table 1. the biochemical indexes of CaPO₄ treated C57BL/6J mice**

| Group | Number | Body weight (g) | Cholesterol(mmol) | Triglyceride(mmol) |
|---|---|---|---|---|
| **NaCl** | **11** | **21.1±1.92** | **3.09±0.37** | **1.22±0.32** |
| **CaPO₄** | **12** | **20.3±1.98** | **3.73±0.93** | **1.22±0.39** |
| **CaPO₄+ NGN** | **11** | **20.1+1.41** | **3.53±0.45** | **0.97±0.22** |
| **CaPO₄+ Dox** | **11** | **21.0±1.89** | **3.49±0.64** | **1.07±0.32** |

In the onset of abdominal aortic aneurysm, inflammatory cell infiltration of vascular walls is a common feature. We used histochemical staining to identify inflammatory cells infiltrated in vascular walls. Mac3 refers to the results of immumohistochemical staining using an anti-Mac3 specific antibody, which is representative for macrophages, and CD45 refers to the results of immumohistochemical staining using an anti-CD45 specific antibody, which is representative for leukocytes. The results were shown in Figure 3A. CaPO₄ daubing can significantly increase the infiltration of macrophages and leukocytes in the adventitia of vascular walls, while a naringenin or doxycycline gavage can reduce this effect.

IL-6 and MCP-1 are proinflammatory chemotactic factors and cytokines, and they are mainly released from vascular walls. MCP-1 can increase the release of matrix metalloproteinases, which degrade the matrix by recruiting leukocytes, thereby mediating vascular remodeling and expanding (MacTaggart JN et al. Deletion of ccr2 but not ccr5 or cxcr3 inhibits aortic aneurysm formation Surgery. 2007; 142:284-288; Ishibashi M et al. Bone marrow-derived monocyte chemoattractant protein-1 receptor ccr2 is critical in angiotensin ii-induced acceleration of atherosclerosis and aneurysm formation in hypercholesterolemic mice. Arterioscl throm vasc biol. 2004; 24: e174-178), while IL-6 can increase the effect of MCP-1, thereby aggravating the onset of abdominal aortic aneurysms (Tieu BC et al. An adventitial il-6/mcp1 amplification loop accelerates macrophage-mediated vascular inflammation leading to aortic dissection in mice. J Clin Invest. 2009; 119:3637-3651). We used histochemical staining to identify the expression of IL-6 and MCP-1 in vascular walls. IL-6 and MCP-1 refer to immunohistochemical results of anti-IL-6 and anti-MCP-1 specific antibodies (purchased from Abcam, Cambridge Company, UK), respectively. The results were shown in Figure 3B. CaPO₄ daubing can significantly increase the expression of IL-6 and MCP-1 in the adventitia of vascular walls, while a naringenin or doxycycline gavage can reduce their expression.

One factor of the pathogenesis of an abdominal aortic aneurysm is the activation of matrix metalloproteinase, which can significantly increase the degradation of the matrix, thereby causing the onset of an abdominal aortic aneurysm. After the aortas of mice were perfused with PBS, fresh tissue was directly embedded with OCT, put in liquid nitrogen for freezing, and then continuously sliced. Firstly, washing was performed three times with PBS for 3 min each. A fluorescently labeled DQ-gelatin-FITC substrate (Sigma-Aldrich, St. Louis, MO) was incubated for 1 hour at 37°C. Under normal conditions, the fluorescent label of the substrate is quenched, such that there is no fluorescence; while activated gelatinase degrades the gelatin, such that the green fluorescent label would be exposed. After the section was incubated, washing was performed three times with PBS for 3 min each, and then the section was sealed. Immunofluorescence confocal microscopy was used for irradiation. The intensity of the fluorescence indicates the activity level of the matrix metalloproteinases. The results of *in situ* zymography in Figure 4A show, that CaPO₄ daubing significantly increases the activity of matrix metalloproteinases, while a naringenin or doxycycline gavage can reduce the activity of matrix metalloproteinases induced by CaPO₄ daubing.

### 3. Gelatin zymography

After freshly removed blood vessels at the infrarenal site in abdominal aortas of mice were cleared from extravasated blood and fat and connective tissues were removed, blood vessels were cut into 1 cm rings, and transferred into a 24-well plate. Blood vessels were cultured for 24 hours using 0.5ml serum-free medium with high glucose. After proteins in the cultured supernatant were quantified, mercaptoethanol-free protein sample loading buffer was added, and the sample was cryopreserved at -80°C. Water was used to prepare gelatin (Sigma-Aldrich, St. Louis, MO) in a concentration of 10mg/ml, which was added to a protein separation gel electrophoresis at the proportion of 1 : 10, resulting in a final concentration of 1mg/ml gelatin. Electrophoresis was performed at a voltage of 80V at 4°C. The gel was transferred into 0.25% Trixon-100 solution, and washing was performed twice in 30 minutes. Then the gelatin was added to an incubation solution (50mM Tris, 0.2M NaCl, 5mM CaCl₂, 0.02% sodium azide) and incubated for 24-48 hours at 37°C. The incubated gel was transferred into Coomassie brilliant blue for staining for 1-2 hours at room temperature, thereafter washed for 30 minutes with a solution containing 30% methanol and 10% glacial acetic acid, a solution having 20% methanol and 10% glacial acetic acid, and a solution having 10% methanol and 5% glacial acetic acid, respectively, and then analyzed with the Odyssey infrared fluorescence scanning system (LI-COR Biosciences, Lincoln, NE). Both MMP-2 and MMP-9 are gelatinases, and can degrade gelatin; whereby the locations where the gelatin is degraded are white, while other locations remain black. Under normal conditions, both MMP-2 and MMP-9 exist in the form of zymogens, but after they are activated, the zymogens are cleaved into activated proteases. Therefore, both MMP-2 and MMP-9 show two white stripes, the upper being the zymogen form, and the lower the activated metalloproteinase. The activated or zymogen form of MMP-2 or MMP-9 can degrade gelatin, the results were shown in Figure 4B, and a naringenin or doxycycline gavage can reverse the increase of MMP-2 and MMP-9 caused by CaPO₄ daubing.

### Example 2. Naringenin reduces the onset of abdominal aortic aneurysms induced by angiotensin II in mice.

Firstly, 16-weeks old male ApoE^{-/-} mice were randomly divided into two groups, a control group (n=16) and a naringenin group (n=15), respectively. Then they were anesthetized by 0.1% pentobarbital sodium, and mini pumps (model 2004, Alzet, Cupertino, CA) were subcutaneously implanted; angiotensin II (Sigma-Aldrich, St. Louis, MO) dissolved with normal saline was added to the pump, such that the drugs can be continuously administrated for 4 weeks at a dosage of 1000ng/kg/min. From the first day after surgery, gavage was daily performed in mice at constant time points, with a water gavage in the control group, and a naringenin gavage (50mg/kg) in the naringenin group. Materials were removed 28 days after gavage. The blood pressure of the mice was measured by the tail cuff method with the BP-89A sphygmomanometer (Beijing Softron Biotechnology Co., Ltd.), and the mice were weighted. Blood was drawn from the inner canthus of the mice, and, after centrifuging it for 10 minutes at 6000 rotations, the supernatant was removed and the blood fat levels of the mice were measured. Naringenin reduces the levels of cholesterol, triglyceride, and low density lipoprotein in mice, which corresponds to the previous report, but it does not have an effect on the blood pressure and the body weight of the mice, see Table 2.

**Table 2. Biochemical indexes of angiotensin II implanted pump induced ApoE^{-/-} knockout mice**

| Group | **AngII** | **AngII + NGN** |
|---|---|---|
| Number | **14** | **14** |
| Body weight (g) | **28.8±1.42** | **28.9±2.03** |
| Cholesterol (mmol) | **12.3±1.36** | **8.46±2.45*** |
| Triglyceride (mmol) | **1.66±0.62** | **0.69±0.35*** |
| Low density lipoprotein(mg/deciliter) | **88.9±45.7** | **44.1±15.9*** |
| High density lipoprotein (mg/deciliter) | **55.3±5.35** | **56.5±5.97** |
| Blood pressure (mmHg) | **131.0±16.20** | **136.6±14,27** |

Aortas of mice were fixed and separated. The Angll group was the control group of angiotensin II implanted pumps and water gavage. Angll + NGN group was the group of angiotensin II implanted pumps and a naringenin gavage. Four blood vessels were randomly selected in each group for photographing. The results were shown in Figure 5A, the first two blood vessels in the Angll group show significant bulges at the suprarenal site, which exceed 50% of the diameters of the blood vessels at the adjacent sites, and arterial aneurysms are formed. However, in the naringenin gavage group, there are arterial aneurysms formed at the suprarenal site only in the first blood vessel, while there are no arterial aneurysms formed in the remaining three blood vessels, showing that the naringenin gavage can reduce the formation of abdominal aortic aneurysms of mice induced by angiotensin II.

Next, the statistics of the onset of abdominal aortic aneurysms in all mice was calculated. In the control group of angiotensin II implanted pump and water gavage, only one of 16 mice died from the fracture of aortic dissection within the first 7 days after implanting the pump, and another one died from the fracture of abdominal aortic aneurysms 1 week after implanting the pump, while abdominal aortic aneurysms were generated in 6 of the remaining 14 mice at the 28th day after implanting the pump. Accordingly, in the control group of angiotensin II implanted pump, aortic aneurysms were formed in 8 of 16 mice, that is, the morbidity rate of aortic aneurysms was 50%. In the group of angiotensin II implanted pump and naringenin gavage, 1 of 15 mice died from the fracture of aortic aneurysms, while only 1 of the remaining mice suffered from abdominal aortic aneurysms at the 28th day after implanting the pump. Accordingly, in the group of angiotensin II implanted pump and naringenin gavage, only two of 15 mice suffered from aortic aneurysms, that is, the morbidity rate of aortic aneurysms was 13.3%. These results show that the naringenin gavage can significantly reduce the onset of angiotensin II mediated aortic aneurysms of mice, see Table 3. The statistical situation of the onset of aortic aneurysms was shown in Figure 5B.

**Table 3. Onset situation of aortic aneurysms mediated by angiotensin II in ApoE^{-/-} mice with water/naringenin gavage**

| Group | Number | Dissection | Fracture | aneurysm | Without aneurysm |
|---|---|---|---|---|---|
| **AngII** | **16** | **1** | **1** | **6** | **8** |
| **AngII+NGN** | **15** | **0** | **1** | **1** | **13** |

### Example 3. Naringenin can reduce the severity of aortic aneurysms

After removing the materials from the mice, their aortas were perfused with 4% paraformaldehyde and fixed, the suprarenal aorta from the diaphragm muscle to the renal artery branch on the right side was equally divided into two segments, which were transferred into liquid nitrogen to freeze for 30 s after embedding with OCT. Serial sections were performed, the thickness of each section was 6 µm, with a spacing of 300 µm, and continuous 16 sections were performed on each mouse. H&E staining was used to determine the diameters of the abdominal aortas of mice, the diameters were measured and the maximum diameter was selected therefrom. The maximum diameter was compared with the diameter of the adjacent site, and if the maximum diameter exceeded 50% of the diameter of the adjacent site, it can be considered that the mouse suffered from an abdominal aortic aneurysm. Figure 6A shows the maximum diameter of suprarenal abdominal aortas of the surviving mice after angiotensin II implanting pump with naringenin or water gavage, wherein each group included 11 mice. The results show that the maximum diameter of the abdominal aortas of mice significantly decreases after administration of a naringenin gavage.

We used Gömöri staining to determine the fracture situation of the elastic lamina of the abdominal aortas of mice, and statistics were performed. The results in Figure 6B show, that the fracture of the elastic lamina of the abdominal aortas of mice decreases after administration of a naringenin gavage, and in each group all 6 mice were included into the analysis. The data in Figure 6 represent average values ± standard deviation.

These results show that a naringenin gavage can significantly reduce the onset and severity of abdominal aortic aneurysm mediated by angiotensin II in mice.

### Example 4. Naringenin gavage can reduce the inflammatory response of blood vessels mediated by angiotensin II in mice

### 1. Naringenin inhibits the inflammatory cell infiltration of vascular walls

CD45 and Mac3 refer to results of an immunofluorescent staining using an anti-Mac3 specific antibody and an anti-CD45 specific antibody, respectively. The results in Figure 7A show that, compared with the control group receiving a water gavage, a naringenin gavage can significantly reduce the infiltration of inflammatory cells (macrophages and leukocytes) in the adventitia.

### 2. Naringenin reduces the levels of IL-6 and MCP-1 in the plasma

Mice were sacrificed by CO₂ inhalation and blood was drawn, 20 µl of heparin was added into the blood, and after centrifuging it at 6000 rpm and 4°C for 10 minutes, the supernatant was aspirated. Ten microliter of supernatant was used, and 10 µl of beads were added, and then 10 µl of PE was added, washed twice with wash buffer after incubating for 2 hours at room temperature, and then 200 µl of wash buffer is added. Sorting was performed on a flow cytometer after each sample was transiently vortexed for resuspension of beads (wherein, the flow cytometry kits for inflammatory factors of mice were purchased from BD Company). The results in Figure 7B show that, after administration of a naringenin gavage, the levels of IL-6 and MCP-1 in the plasma decrease significantly (n=11, P <0.05). In contrast, II-10, an anti-inflammatory factor, does not change significantly, while TNF-α and IFN-γ do not change, either.

### 3. Naringenin reduces the expression of IL-6 and MCP-1 in vascular walls

In order to illustrate the mechanism by which naringenin reduces the inflammatory reaction of abdominal aorta vascular walls of mice, immunofluorescent staining was performed on IL-6 and MCP-1 by using continuous sections to analyze their distributions and expression in the vascular walls. Confocal fluorescence microscope was used to irradiate, green light indicated IL-6 or MCP-1, and blue light indicated the cell nucleus. The results in Figure 7C show that, when a naringenin gavage is administered to mice, the production of IL-6 and MCP-1 in the vascular walls decreases significantly, illustrating that naringenin can reduce the contents of IL-6 and MCP-1 in vascular walls.

### 4. Naringenin gavage reduces the activity and expression of metalloproteinase in mice

The results in Figure 8A show that, after administering angiotensin II, the activity of the metalloproteinases in the mesolamella and the adventitia is high, while after administration of a naringenin gavage, the activated metalloproteinases decrease significantly.

The results in Figure 8B show that, after using a naringenin gavage, the zymogen form of MMP-9 decreases significantly, that is, naringenin inhibits the synthesis of MMP-9, while the active forms of MMP-9 and MMP-2 do not change significantly.

### Example 5. The effect of naringenin on the development and outcome of the generated abdominal aortic aneurysms

The CaPO₄ daubed mouse model and the angiotensin II induced mouse model, respectively, were used to investigate whether naringenin affects aortic aneurysms once they were formed, or not.

In the CaPO₄ model, we daubed CaPO₄ at the infrarenal abdominal aortas in 12-week old of male C57 mice (totally 36 mice), and arterial aneurysms were formed 7 days later. Next, the 36 mice were randomly divided into 2 groups: one group was given a naringenin (50 mg/kg/day) gavage, and another group was given a water gavage in an equal manner. Gavage was performed for 2 weeks (8 mice for each group) and 4 weeks (10 mice for each group), respectively. We found by ex vivo measuring method (the results were shown in Figure 9), that, compared with the control group, naringenin can significantly inhibit the progression of arterial aneurysms and reduce the average diameter of infrarenal abdominal aortas (2 weeks, 0.884±0.33 vs. 1.488±0.42 mm, ***P***<0.05; and 4 weeks, 0.9935±0.5 vs.1.718±0.64 mm, ***P***<0.05).

In addition, we used the method of angiotensin II subcutaneous implanted pump for 28 days (1,000 ng/kg/min) to induce abdominal aortic aneurysms on 20-weeks old male ApoE knockout mice (totally 40 mice), wherein ultrasound was used to detect the diameters of the abdominal aortas. A diameter larger than 50% of the diameter of the adjacent blood vessels was used as a standard for the onset of an aortic aneurysm, and 28 mice suffered from aortic aneurysms 28 days later. A similar maximum abdominal aorta diameter was used as grouping standard, and we equally divided the mice which suffered from aortic aneurysms (28 mice) and did not suffer from aortic aneurysm (12 mice) into two groups, the average maximum abdominal aorta diameters were equal; one group was given a naringenin gavage, and the other group was given a water gavage in an equal manner. An angiotensin II implanted pump was continuously given to all the mice for 28 days. The same results as in the CaPO₄ model were observed: in mice which did not suffer from aortic aneurysms previously and received a naringenin gavage, only one of 6 mice suffered from aortic aneurysms 28 days later (with the incidence rate of 16%), while 3 mice in the control group suffered from aortic aneurysms (with the incidence rate of 50%). In mice which had suffered from aortic aneurysms previously and received a water gavage, 4 out of 14 mice died from fracture of aortic aneurysms, and the other 10 surviving mice all suffered from serious aortic aneurysms (the average diameter reaches 3.00±0.58 mm). In contrast, after the second round of angiotensin II simulation, the mice in the naringenin gavage group all survived. In these 14 mice, aortic aneurysms were observed in 5 mice only, and the aortic aneurysms in the remaining mice were all reversed, and the maximum diameter of the blood vessels was reduced to 1.62±0.45 mm. In addition, we found by using gelatin zymography to detect the activity of metalloproteinase, that naringenin can significantly inhibit the activity of metalloproteinase MMP-9.

In summary, naringenin cannot only inhibit the formation of abdominal aortic aneurysms, but can also reverse aortic aneurysms after they formed, such that a therapeutic effect is exerted.

### Industrial Application

The drugs for preventing and/or treating abdominal aortic aneurysms provided by the present invention are safe and low toxic, and the pharmacologic effects thereof are strong; sources of the raw materials thereof are rich, wide, inexpensive, and can be obtained from crude products or from monomers of naringenin by a hydrolysis method, or it can be obtained by extracting it from various crude medicines containing naringenin, or it can be obtained by using other chemical synthesis methods; the costs are low, the process is simple, and the yield is high; the curative effect is definite, and the effective dose is low. A new drug source for preventing, diagnosing, detecting, protecting, treating, and studying abdominal aortic aneurysm diseases is described herein, which is easy to popularize and apply, and which can produce huge social and economic benefits within a short period of time.

## Claims

1. Naringenin for use in a method of preventing and/or treating abdominal aortic aneurysms.

2. Naringenin for the use of claim 1, wherein the prevention and/or treatment of abdominal aortic aneurysms comprises:
1) inhibiting the fracture of abdominal aorta elastic lamina;
2) inhibiting the infiltration of inflammatory cells of abdominal aorta vascular walls;
3) inhibiting the levels of interleukin IL-6 and monocyte chemotactic factor MCP-1 in the plasma;
4) inhibiting the expression of inflammatory factors in the abdominal aorta vascular walls; and/or
5) inhibiting the activity and expression of matrix metalloproteinases in abdominal aorta vascular walls.

3. Naringenin for the use of claim 2, **characterized in that** the inflammatory factors are interleukin IL-6 and monocyte chemotactic factor MCP-1.

4. Naringenin for the use of claim 2, **characterized in that**: the inflammatory cells are macrophages and/or leukocytes.

5. Naringenin for the use of claim 2, **characterized in that**: the matrix metalloproteinase is MMP-9.

6. A pharmaceutical composition comprising naringenin as an active ingredient for use in a method of preventing and/or treating abdominal aortic aneurysms.

## Patentansprüche

1. Naringenin zur Verwendung in einem Verfahren zur Vorbeugung und/oder Behandlung von abdominalen Aortenaneurysmen.

2. Naringenin zur Verwendung nach Anspruch 1, wobei die Prävention und/oder Behandlung von abdominalen Aortenaneurysmen umfasst:
1) Hemmung der Fraktur der abdominalen elastische Lamina der Aorta;
2) Hemmung der Infiltration von Entzündungszellen der Gefäßwände der abdominalen Aorta;
3) Hemmung der Spiegel von Interleukin IL-6 und des Monozyten-chemotaktischen Faktors MCP-1 im Plasma;
4) Hemmung der Expression von Entzündungsfaktoren in den Gefäßwänden der abdominalen Aorta; und/oder
5) Hemmung der Aktivität und Expression von Matrix-Metalloproteinasen in den Gefäßwänden der abdominalen Aorta.

3. Naringenin zur Verwendung nach Anspruch 2, **dadurch gekennzeichnet, dass** die Entzündungsfaktoren Interleukin IL-6 und der Monozyten-chemotaktische Faktor MCP-1 sind.

4. Naringenin zur Verwendung nach Anspruch 2, **dadurch gekennzeichnet, dass** die Entzündungszellen Makrophagen und/oder Leukozyten sind.

5. Naringenin zur Verwendung nach Anspruch 2, **dadurch gekennzeichnet, dass**: die Matrix-Metalloproteinase MMP-9 ist.

6. Eine pharmazeutische Zusammensetzung, umfassend Naringenin als Wirkstoff zur Verwendung in einem Verfahren zur Vorbeugung und/oder Behandlung von abdominalen Aortenaneurysmen.

## Revendications

1. Naringénine pour l'utilisation dans un procédé de prévention et/ou de traitement des anévrismes de l'aorte abdominale.

2. Naringénine pour l'utilisation selon la revendication 1, la prévention et/ou le traitement des anévrismes de l'aorte abdominale comprenant :
1) l'inhibition de la fracture de la lame élastique de l'aorte abdominale ;
2) l'inhibition de l'infiltration de cellules inflammatoires des parois vasculaires de l'aorte abdominale ;
3) l'inhibition des niveaux d'interleukine IL-6 et du facteur chimiotactique des monocytes MCP-1 dans le plasma ;
4) l'inhibition de l'expression des facteurs inflammatoires dans les parois vasculaires de l'aorte abdominale ; et/ou
5) l'inhibition de l'activité et de l'expression des métalloprotéinases de la matrice dans les parois vasculaires de l'aorte abdominale.

3. Naringénine pour l'utilisation selon la revendication 2, **caractérisée en ce que** les facteurs inflammatoires sont l'interleukine IL-6 et le facteur chimiotactique des monocytes MCP-1.

4. Naringénine pour l'utilisation selon la revendication 2, **caractérisée en ce que** : les cellules inflammatoires sont des macrophages et/ou des leucocytes.

5. Naringénine pour l'utilisation selon la revendication 2, **caractérisée en ce que** : la métalloprotéinase de la matrice est la MMP-9.

6. Composition pharmaceutique comprenant de la naringénine comme ingrédient actif pour l'utilisation dans un procédé de prévention et/ou de traitement des anévrismes de l'aorte abdominale.
